# EUROPEAN PATENT APPLICATION

(11) **EP 1 297 820 A1**
(43) Date of publication of application: **02.04.2003**
(21) Application number: 01945786.0
(22) Date of filing: 03.07.2001
(51) Int. Cl.: A61K 7/32, A61K 7/00, A61K 7/06

(54) **DEODORANT COMPOSITIONS**

(30) Priority: 03.07.2000 JP 2000200612; 08.12.2000 JP 2000374106
(71) Applicant: Kao Corporation, Tokyo 103-8210 (JP)
(72) Inventor: TSUCHIKURA, Keiji, C/O Kao Corporation Res. Lab., Tokyo 131-8501 (JP)
(74) Representative: HOFFMANN - EITLE
(86) International application number: JP0105749
(87) International publication number: WO02002064

(57) **Abstract**

Provided is a deodorant having excellent masking and deodorizing effects of scalp odor, body odor and foot odor. This deodorant comprises a betaine ester represented by the following formula (1): 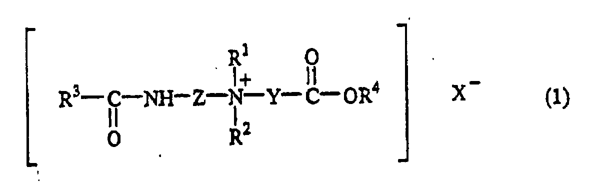 [wherein, R¹, R² and R³ each independently represents a hydrogen atom or a linear or branched C₁₋₃₆ alkyl or alkenyl group, Y represents a linear or branched C₁₋₄ alkylene group, Z represents a group represented by -R⁵(OA)ₙ- (in which, R⁵ represents a linear or branched C₁₋₃₆ alkylene group, A represents a linear or branched C₂₋₄ alkylene group and n stands for 0 to 10), R⁴ represents a residue left after removal of one OH from an alcohol compound having, in the molecule thereof, at least one hydroxyl group, and X⁻ represents an anion].

## Description

### Technical Field

The present invention relates to deodorants excellent in masking and deodorization of a scalp odor, body odor and foot odor; and hair cosmetic compositions and body cosmetic compositions containing them.

### Background Art

In daily life, people tend to sweat much particularly upon attending office or school in crowded trains, on a hot day or in a state of nervous tension, which deprives them of a comfortable state or gives an unpleasant impression to the nearby people. In addition to unpleasantness in wet clothes or stockings, an offensive odor such as body odor or foot odor which starts from perspiration and lasts even after drying of the clothes is a more important cause for the above-described discomfort. The body odor includes an acid odor such as acetic acid and isovaleric acid and a fat odor such as long-chain fatty acid, while the offensive foot odor includes an acid odor such as acetic acid and isovaleric acid, a fat odor such as long-chain fatty acid, an ammonia odor, an odor of amines relating a stinky foot odor promoted under wet and warm conditions and an N-compound odor such as amino acid. Among these body odors and foot odors, the fat odor and the stinky foot odor have a high threshold, but they hold a large proportion in the composition of the body odor and foot odor. Combination of the fat odor and the stinky odor plays a base-note like role in perfumery compared with the acid odor and the ammonia odor and increases the whole unpleasant odor. An agent for masking or deodorizing such a combination of fatty odor and stinky odor, however, has not been proposed yet.

As another body odor, there exists a scalp odor. The scalp odor is composed of three elements such as acid odor, fat odor and stinky odor promoted under wet and warm conditions. An agent effectively deodorizing them has not yet been proposed so far.

The activity of indigenous bacterial on the skin is one of the causes for body odor, foot odor and scalp odor. Direct use of an antibacterial agent or bactericide or incorporation of it in an antiperspirant or foot spray for the purpose of suppressing the activity of these bacteria needs a deliberate care so as not to inhibit sound physiological function or metabolic function of the human body, leading to a problem such as limitation in its using amount.

With regards to the body odor and foot odor, in addition, it is the common practice to mask the body odor or foot odor with a perfume such as Eau de Parfum, Eau de Toilette, Eau de Cologne, antiperspirant or foot spray. This method is however accompanied with the drawback that Eau de Palfam, Eau de Toilette, Eau de Cologne, antiperspirant or foot spray applied to the skin mixes with the body odor or foot odor, thereby preventing sufficient elimination of the odor; or Eau de Palfum, Eau de Toilette, Eau de Cologne, antiperspirant or foot spray having a strong smell enough for masking the body odor or foot odor sometimes make others uncomfortable.

With regards to the scalp odor, it is the common practice to mask it with a perfume contained in a hair cologne or styling agent. This method is also accompanied with the drawback that the smell of the hair cologne or styling agent mixes with the scalp odor, thereby preventing sufficient elimination of it; or a hair cologne having a strong smell enough for masking the scalp odor sometimes make others uncomfortable.

An object of the present invention is to provide a deodorant excellent in masking and deodorizing effects of body odor, foot odor and scalp odor; and a hair cosmetic composition and a body cosmetic composition containing it.

### Disclosure of the Invention

The present inventors have found that a betaine ester having a specific structure has excellent masking and deodorizing effects of body odor, foot odor and scalp odor.

In the present invention, there is thus provided a deodorant comprising a betaine ester represented by the following formula (1): 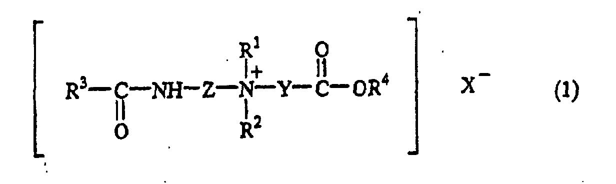 [wherein, R¹, R² and R³ each independently represents a hydrogen atom or a linear or branched C₁₋₃₆ alkyl or alkenyl group, Y represents a linear or branched C₁₋₄ alkylene group, Z represents a group represented by -R⁵(OA)ₙ- (in which, R⁵ represents a linear or branched C₁₋₃₆ alkylene group, A represents a linear or branched C₂₋₄ alkylene group and n stands for 0 to 10), R⁴ represents a residue left after removal of OH from an alcohol compound having, in the molecule thereof, at least one hydroxyl group, and X⁻ represents an anion].

In another aspect of the present invention, there is also provided a hair cosmetic composition or body cosmetic composition containing a betaine ester represented by the formula (1) as a deodorant.

### Best Mode for Carrying Out the Invention

In the present invention, a betaine ester represented by the formula (1) is used. In the formula (1), the alkyl, alkenyl and alkenylene groups may each have a substituent. Examples of the substituent include aryl groups such as phenyl, alkoxy groups such as methoxy and ethoxy and aryloxy groups such as phenoxy.

The betaine ester is available, for example, by reacting a compound represented by the formula (3) (which will hereinafter be called "Compound (3)" simply) with a compound represented by the formula (4) (which will hereinafter be called "Compound (4)", simply) in the presence of a solvent. 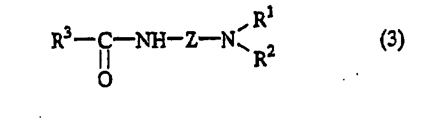 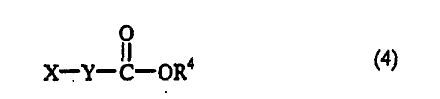 [wherein, R¹, R², R³, R⁴, X, Y And Z have the same meanings as described above].

As the solvent, diethyl ether, acetone or chloroform is employed, depending on the solubility of the raw materials. The reaction temperature can be selected within a range of 15 to 100°C, depending on the boiling point of the solvent. Preferred is 25 to 50°C, with 40 to 45°C being particularly preferred. A charging ratio of Compound (3) to (4) is preferably set so that the amount of Compound (4) is used in excess in order to increase a reaction ratio and facilitates purification of the product. From the practical viewpoint, a molar ratio within a range of from 1:1 to 1:2 is preferred.

Among the betaine esters, a compound represented by the formula (2) (which will hereinafter be called "betaine ester (2)") is particularly preferred. 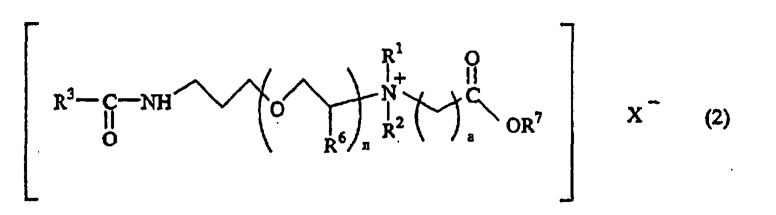 [wherein, R¹, R², R³, n and X⁻ have the same meanings as described above, R⁶ represents a hydrogen atom or a methyl group, a stands for 1 or 2, R⁷ represents a saturated or unsaturated aliphatic hydrocarbon or aromatic hydrocarbon group having 1 to 11 carbon atoms, or a C₁₂₋₃₆ alkadienyl, alkatrienyl, aryl or arylalkyl group, or a monocyclic, dicyclic or tricyclic terpene hydrocarbon group, some of hydrogen atoms on said hydrocarbon group of R⁷ may be substituted or unsubstituted by a halogen atom or a hydroxyl group, the methylene group in said hydrocarbon group of R⁷ may be substituted or unsubstituted by a carbonyl group, an amide bond, an oxygen atom or a sulfur atom, the methyl group may be substituted or unsubstituted by a formyl group or -CONH₂, the carbon-carbon double bond may be substituted or unsubstituted by an epoxy group, or R⁷ may be either a single substance or a mixture].

In the betaine ester (2), R¹ and R⁴ each preferably has 1 to 4 carbon atoms, with a methyl group being particularly preferred. R³ preferably has 1 to 22 carbon atoms, while n is preferably 0 to 3. Examples of the anion represented by X⁻ include halogen ions, sulfuric acid ions, bisulfate ions and perchlorate ions. Of these, chlorine ions are preferred. The followings are specific examples of the group represented by R⁷.

Examples include residues left after removal of one hydroxyl group from 1-menthol, d-menthol, 1-isopulegol, d-isopulegol, 1-piperitol, d-piperitol, (+)-cis-Thujan-4-ol, (+)-trans-Thujan-4-ol, ethanol, cis-3-hexenol, benzyl alcohol, borneol, thymol, hinokitiol, capsaicin, farnesol, triclosan or cis-3-hexenol.

As the alcohol compounds before removal of one hydroxyl group, primary or secondary ones are particularly preferred among them from the viewpoint of the stability. Preferred ones include 1-menthol, d-menthol, 1-isopulegol, d-isopulegol, 1-piperitol, d-piperitol, (+)-cis-Thujan-4-ol, (+)-trans-Thujan-4-ol, ethanol, and cis-3-hexenol.

The betaine ester of the formula (1) usable in the present invention can be used as is as a deodorant. Alternatively, components ordinarily employed for a deodorant composition may be used in combination as needed. In this case, the content of the betaine ester represented by the formula (1) in the deodorant composition is 30 to 100 wt.% (which will hereinafter be called "%", simply), preferably 50 to 100%, especially 70 to 100%. Addition of a functional perfume is preferred to reinforce the deodorizing effects. The term "functional perfume" as used herein means a perfume material having deodorization reinforcing effects when used in combination with a deodorizing base. The functional perfume can be used singly. More specifically, the functional perfumes as exemplified later in the hair cosmetic composition or body cosmetic composition are preferred. The content of it in the deodorizing composition is 0.001 to 70%, preferably 0.005 to 50%, especially 0.01 to 30%.

The deodorant composition may further contain a diluent such as propylene glycol or diethyl phthalate and an antibacterial agent such as benzalkonium chloride or benzethonium chloride.

The deodorant composition of the present invention is prepared by dissolving and dispersing the above-described components in a solvent such as water, ethanol, liquid paraffin, benzyl alcohol, benzyloxyethanol, menthol, limonene, "EXCEPARL" (product of Kao Corp) or "EXCEL" (fatty acid monoglyceride, product of Kao Corp.), or a surfactant, particularly in "EXCEPARL", ethanol or menthol.

In the case of the hair cosmetic composition containing a betaine ester of the formula (1) as a deodorant, the content of the betaine ester is 0.01 to 70%, preferably 0.01 to 50%, especially 0.01 to 30%.

Examples of the functional perfume excellent in deodorization reinforcing effects and masking effects of a scalp odor include α-pinene, β-pinene, methyl anthranilate, isobutyl quinoline, eugenol, aldehyde C-10, coumarin, vanillin, triplal, cis-3-hexenol, α-ionone, β-ionone, γ-ionone, α-isomethyl ionone, allyl ionone, α-methyl ionone, β-methyl ionone, γ-methyl ionone, α-irone, β-irone, γ-irone, methylionone-γ, sandal mysore core, aldehyde-C-14 peach, α-damascone, β-damascone, γ-damascone, α-dynascone, β-dynascone, lilial, tuberose, calanal, ambroxan, p-cresol, maracuja, moscyns, olibanum resinoid, gelanyl nitrile, phenoxyethyl alcohol, fluloza, heliotropin, anisyl acetate, anisylacetone, acetyl eugenol, acetyl isoeugenol, pentalide and cyclohexyl salicylate.

Functional perfumes such as 1-carbon, geraniol, citral, thymol, 1,8-cineol, peppermint oil, spearmint oil, lemon grass oil and cedar leaf oil have antibacterial action so that they are preferred as a perfume for suppressing generation of an offensive odor due to proliferation of bacteria on the lipid or sweat components attached to the hair.

No particular limitation is imposed on the perfume used for scenting of the hair cosmetic composition of the present invention. At least one of the above-exemplified functional perfumes may be incorporated in the composition. Its content is preferably 0.001 to 30%, especially 0.001 to 5% in the whole hair cosmetic composition.

Functional perfumes and perfumes for scenting may be diluted with a diluent. Use, as the diluent, of at least one compound selected from 2-methyl-2,4-dihydroxybutane, 2-methyl-2,4-dihydroxypentane, 2,4-dihydroxybutane, propylene glycol, dipropylene glycol, tripropylene glycol, ethanol, liquid paraffin, 3-methoxy-3-methylbutanol, diethyl phthalate and IP solvent is preferred.

The hair cosmetic composition may contain, in addition to the functional perfume having an antibacterial action, an antibacterial agent such as zinc pyrithione (zinc 2-pyridinethiol-1-oxide), aluminum chloride, zinc oxide, isopropylmethylphenol, benzalkonium chloride, triclosan, chlorhexidine chloride, halocalvan, benzethonium chloride, chlorhydroxy aluminum, allantoin chlorxyaluminum or zinc p-phenolsulfonate in an amount of 0.001 to 10%, preferably 0.01 to 1%.

In case of the hair cosmetic composition of the present invention containing the betaine ester of the formula (1) as a deodorant composition, the composition is added in an amount of 0.01 to 50%, preferably 0.01 to 20%.

In the case of the body cosmetic composition of the present invention containing the betaine ester of the formula (1) as a deodorant, the betaine ester is preferably added in an amount of 0.01 to 70%, preferably 0.01 to 50%, especially 0.01 to 30%.

It is preferred to add the functional perfume to the body cosmetic composition of the present invention because it can reinforce deodorizing effects further. The content of the functional perfume in the body cosmetic composition is 0.001 to 70%, preferably 0.005 to 50%, especially 0.01 to 30%.

As the functional perfume having masking effects of body odor and foot odor, γ-undecalactone, magnol and musk ketone are most preferred, while α-damascone (β, γ), ethyl tricyclo[5.2.1.02,6]decane-2-carboxylate, γ-decalactone, δ-decalactone, γ-octalactone, γ-nonalactone, dynascone, helional, cis-3-hexenyl salicylate, eurantiol, benzyl salicylate, α-hexylcinnamic aldehyde, methyl dihydrojasmonate, methyl jasmonate, methylionone-γ, α-, β-, γ-ionone, isocanphil cyclohexanol (sandalsins), 2-methyl-4-(2,3,3-triethyl)-3-cyclopenten-1-yl)-2-buten-1-ole, 3α,6,6,9α-tetramethyldodecahydronaphtho[2,1-b]furan, indole, indolene, ethylene brassylate are also preferred. Lauryl aldehyde, decatone, lemonile, undecarbitol, floralozone, eugenol, iso-eugenol, 7-acetyl-1,2,3,4,5,6,7,8-octahydro-1,1,6,7-tetramethylnaphthalene, 1-(2-t-butylcyclohexyloxy)-2-butanol, 6-acetylhexatetralin and cyclopentadecanolide are usable.

In the body cosmetic composition of the present invention, at least one perfume included in the functional perfumes may be incorporated as a scenting perfume. Its content in the whole body cosmetic composition is preferably 0.001 to 30%, especially 0.001 to 5%.

The perfume usable as a functional perfume or perfume for scenting may be diluted with a similar diluent to that exemplified in the hair cosmetic composition. In the body cosmetic composition of the present invention, an antibacterial agent similar to that used for the hair cosmetic composition can be incorporated, in addition to the functional perfume having an antibacterial action. The content of the antibacterial agent in the body cosmetic composition of the present invention may be 0.001 to 10%, preferably 0.01 to 1%.

The hair cosmetic composition or body cosmetic composition of the present invention is prepared by dissolving and dispersing the above-described components in a solvent such as water, ethanol, liquid paraffin, benzyl alcohol, benzyloxyethanol, menthol, limonene, "EXCEPARL" or "EXCEL" (fatty acid monoglyceride), or a surfactant. Use of EXCEPARL, ethanol or menthol as a solvent is especially preferred.

The hair cosmetic composition or body cosmetic composition of the present invention may further contain a surfactant. The surfactant is added to the hair cosmetic composition for an improvement of hair setting effects or exhibition of sufficient cleansing effects. Moreover, since it is effective for preventing an offensive odor due to cigarette, cooking or sweat from hanging in the air, the body cosmetic composition used as a body shampoo exhibits effects for preventing an offensive odor such as cigarette or cooking odor attached to the body from being released from the body as well as sufficient cleansing effects. Addition of it is therefore preferred. Specific examples of the surfactant are as follows. Anionic surfactants usable here include glycolic acid N-alkyl(or alkenyl)amide sulfate ester salts, alkylbenzene sulfonate salts, polyoxyethylene alkyl (or alkenyl) ether sulfate salts, alkyl (or alkenyl) sulfate salts, olefin sulfonate salts, alkanesulfonate salts, saturated or unsaturated fatty acid salts, alkyl (or alkenyl) ether carboxylate salts, α-sulfonate salts or esters, N-acylamino acid type surfactants, mono- or di-phosphate type surfactants, and sulfosuccinate esters.

Nonionic surfactants usable here include polyoxyethylene alkyl (or alkenyl) ethers, polyoxyalkyl phenyl ethers, polyoxypropylene alkyl (or alkenyl) ethers, higher fatty acid alkanolamides or alkylene oxide adducts thereof, sucrose fatty acid esters and glycerin fatty acid monoesters. Of these, polyoxyethylene (1 to 30) lauryl ethers and glycerin fatty acid monoesters are preferred because they are free from thickening action.

Cationic surfactants usable here include quaternary ammonium salts, with mono(long chain) type and di(long chain) type quaternary ammonium salts are preferred. Specific examples of the mono(long chain) or di(long chain) include alkyl, alkenyl, hydroxyalkyl, alkylcarbonylaminoalkyl, alkenylcarbonylaminoalkyl, alkylaminocarbonylalkyl, alkenylaminocarbonylalkyl, alkoxyalkyl, alkenyloxyalkyl, aliphatic acyloxyalkyl, alkoxycarbonylalkyl and alkenyloxycarbonylalkyl groups. The alkyl or alkenyl portion of them may be linear or branched. More specific examples include quaternary ammonium salts usable for the hair cosmetic compositions or softeners as described in Japanese Patent Application Laid-Open No. Sho 62-141176, Sho 63-50574, Sho 63-260991, Sho 63-260992, Sho 63-295765, Hei 7-90773, Hei 7-309723 or Hei 7-309724. Preferred examples include mono(long chain) (linear or branched) alkyltrimethylammonium salts, di(long chain) (linear or branched )alkyldimethylbenzylammonium salts, and di(long chain) (linear or branched) alkylmethylhydroxyethylammonium salts.

Amphoteric surfactants usable here include imidazoline, amide amino acid salts, carbobetaine, alkylbetaine, alkylamidobetaine and alkylsulfobetaine type.

At least two of these surfactants may be used in combination. Addition in an amount of 0.05 to 30%, especially 0.1 to 10% to the hair cosmetic composition or body cosmetic composition is preferred from the viewpoint of lathering.

The hair cosmetic composition or body cosmetic composition of the invention may contain a silicone derivative to improve touch feel and enduring property of the perfume. Such silicone derivatives include dimethylpolysiloxane, methylphenyl siloxane, methylphenyl polysiloxane, amino-modified silicone, polyether-modified silicone, fatty acid modified silicone, fluorine type modified silicone, cyclic silicone, alkyl-modified silicone, alcohol-modified silicone, aliphatic modified silicone and epoxy-modified silicone.

At least two of these silicone derivatives may be used. Their content in the hair cosmetic or body cosmetic composition is preferably 0.01 to 15%, with 0.1 to 10% being especially preferred.

The hair cosmetic composition of the present invention may further contain a cationic polymer to improve smoothness and scenting property of the perfume. Such cationic polymers include cationated cellulose derivatives, cationic starches, cationated guar gum derivatives, diallyl quaternary ammonium salt polymers, diallyl quaternary ammonium salt/acrylamide copolymers and quaternized polyvinylpyrrolidone derivatives.

Such a cationic polymer is preferably added in an amount of 0.01 to 30% to the hair cosmetic composition, with 0.1 to 15% being especially preferred.

The hair cosmetic or body cosmetic composition of the present invention is preferably used in the liquid, powdery, gel or granular form as needed. A liquid form dissolved in a solvent such as water or a lower alcohol, preferably an aqueous lower alcohol solvent, more preferably an aqueous ethanol solution is preferred, because the composition effectively exhibits deodorizing effects in such a form.

The deodorant, deodorizing composition and hair cosmetic composition according to the present invention are preferably employed as leave-on hair care products such as styling agent, hair growth tonic, hair tonic, hair liquid, hair cologne or Eau de Cologne. In the case where the deodorant of the present invention is cationic and a sufficient amount of it remains on the hair even after washed away, they can be used preferably as a hair shampoo, hair rinse, hair conditioner and hair treatment.

The body cosmetic composition of the present invention can be used preferably as a foot spray, skin cream, body lotion, antiperspirant, systemic body shampoo, soap, bath agent, Eau de Palfum, Eau de Toilette, Eau de Cologne or the like.

### Examples

### Synthesis Example 1: N,N-Dimethyl-N-L-menthyloxycarbonylmethyl-N-(3-(octadecanoylamino)propyl)ammonium chloride (betaine ester)

In a 1L four-necked round flask were charged 110.0 g (0.298 mole) of N-octadecanoyl-N',N'-dimethyl-1,3-diaminopropane, 76.38 g (0.328 mole) of L-menthyl chloroacetate and 450 mL of acetone. The mixture was heated under reflux for 6 hours. Substantial disappearance of chloroacetate was confirmed by ¹H-NMR. The reaction mixture was allowed to stand at room temperature for 12 hours. Ice cooling was then conducted to precipitate a white plasma. The supernatant solvent was removed by decantation. An operation of adding 30 mL of acetone, dissolving the residue therein by heating to 55°C, icecooling the solution while stirring and decanting thus precipitated crystals was repeated several times for purification. By drying under reduced pressure by a vacuum pump, a betaine ester (1) was obtained as white crystals in an amount of 114.82 g (0.191 mole; yield: 64.0%).
¹H-NMR (200MHz, CDCl₃): δ[ppm]
0.75 (3H, d, J=6.9Hz, >CH-CH₃), 0.90-1.00 (9H, (CH₃)₂CH-, CH₃-(CH₂)₁₉-), 1.00-1.20 (2H, >CH₂), 1.25 (30H, brs, - (CH₂)₁₅-Me), 1.4-1.9 (7H, cyclic>CH₂, >CH, -CH, -CH₂-CH₂-CONH-), 1.9-2.2 (3H, >CH, N-C-CH₂-C-N), 2.20 (2H, t, J=10.0Hz, -CH₂-CH₂-C=O), 3.36 (2H, dt, J=5.5,7.2Hz, -CONH-CH₂-C-), 3.50 (3H, s, CH₃-N⁺-CH₃), 3.52 (3H, s, CH₃-N⁺-CH₃), 4.13 (2H, dd, J=5.5Hz, -CH₂-CH₂-N⁺-), 4.43 (1H, d, N⁺-CH₂-C=O, J=16Hz), 4.62 (1H, d, N⁺-CH₂-C=O, J=16Hz), 4.82 (1H, td, J=4.5,10.9Hz, >CH-O-C=O), 8.10 (1H, t, J=5.5Hz, -CONH-)
IR (NaCl) [cm⁻¹]
3700-3150, 2958 (νₐₛCH₃), 2920 (νₐₛCH₂), 2850 (νₐₛCH₂), 1736 (νC=O ester), 1655 (νC=O, amide), 1552 (δNH, νC-N), 1467 (δₐₛCH₃; δCH, -CH₂-), 1408 (gem-dimethyl), 1375 (gem-dimethyl), 1227, 1205 (νC-O, ester), 1155, 1020, 955, 918, 721 ((CH₂)ₙ)

### Synthesis Example 2: N-Dimethyl-N-L-menthyloxycarbonylmethyl-N-(3-(docosanoylamino)propyl)ammonium chloride (betaine ester (2))

In a 1L four-necked round flask were charged 3.51 g (0.168 mole) of N-docosadecanoyl-N',N'-dimethyl-1,3-diaminopropane, 46.78 g (0.201 mole) of L-menthyl chloroacetate and 200 mL of toluene. The mixture was heated at 80°C for 2 hours. Substantial disappearance of chloroacetate was confirmed by ¹H-NMR. The solvent was distilled off under reduced pressure by an evaporator. To the crude product was added 500 mL of acetone and the mixture was heated to 55°C to dissolve the former in the latter. While stirring, the solution was air cooled and the crystals thus precipitated were filtered under pressure. These white crystals were collected in the flask again and 500 mL of acetone was added, followed by the same operation 6 times to remove the L-menthyl chloroacetate. By drying under reduced pressure by a vacuum pump, the betaine ester (2) was obtained as white crystals in an amount of 68.3 g (0.102 mole; yield: 60.7%).
¹H-NMR (200MHz, CDCl₃) : δ [ppm]
0.75 (3H, d, J=6.9Hz, >CH-CH₃), 0.90-1.00 (9H, (CH₃)₂CH-, CH₃-(CH₂)₁₉-), 1.00-1.20 (2H, >CH₂), 1.25 (38H, brs, - (CH₂)₁₉-Me), 1.4-1.9 (7H, cyclic>CH₂, >CH, -CH, -CH₂-CH₂-CONH-), 1.9-2.2 (3H, >CH, N-C-CH₂-C-N), 2.20 (2H, t, J=10.0Hz, -CH₂-CH₂-C=O), 3.36 (2H, dt, J=5.5,7.2Hz, -CONH-CH₂-C-), 3.50 (3H, s, CH₃-N⁺-CH₃), 3.52 (3H, s, CH₃-N⁺-CH₃), 4.13 (2H, dd, J=5.5Hz, -CH₂-CH₂-N⁺-), 4.43 (1H, d, N⁺-CH₂-C=O, J=16Hz), 4.62 (1H, d, N⁺-CH₂-C=O, J=16Hz), 4.82 (1H, td, J=4.5,10.9Hz, >CH-O-C=O), 8.10 (1H, t, J=5.5Hz, -CONH-)
IR (NaCl) [cm⁻¹]
3700-3150, 2958 (νₐₛCH₃), 2920 (νₐₛCH₂), 2850 (νₐₛCH₂), 1736 (νC=O, ester), 1655 (νC=O, amide), 1552 (δNH, νC-N), 1467 (δₐₛCH₃; δCH, -CH₂-), 1408 (gem-dimethyl), 1375 (gem-dimethyl), 1227, 1205 (νC-O, ester), 1155, 1020, 955, 918, 721 ((CH₂)ₙ)

The deodorizing effects of the present invention were evaluated by the following method.

### Masking effects of scalp odor

To about 21 g (15 cm) of a false hair bundle sprayed (spray: "Mark II Spray K-24-410") once with a model scalp odor as shown in Table 1, a deodorant composition or hair cosmetic composition was sprayed once (Spray: "Mark II Spray K-24-410") or applied in an amount of 1 g. Masking effects rightly, 30 minutes, one hour and one day after it was spread over the whole hair by a brush were evaluated based on the below-described criteria with the model scalp odor as 5 and its average scores was determined. The average score of 1 or greater but less than 2 was ranked A, that of 2 or greater but less than 3 was ranked B, that of 3 or greater but less than 4 was ranked C, and that of 4 or greater was ranked D.
1: The scalp odor is masked completely.
2: The scalp odor is substantially masked.
3: A slight scalp odor remains.
4: A scalp odor remains (masking is a little insufficient).
5: A scalp odor evidently remains (masking is insufficient).

**Table 1**

| | Component % |
|---|---|
| Proline | 0.007 |
| Leucine | 0.018 |
| Phenylalanine | 0.007 |
| Serine | 0.034 |
| Threonine | 0.016 |
| Citrulline | 0.001 |
| Valine | 0.009 |
| Arginine | 0.0045 |
| Lysine | 0.0045 |
| Pyrrolidine | 0.001 |
| Isobutylamine | 0.001 |
| Piperidine | 0.001 |
| Ammonia | 0.001 |
| Methyl mercaptan | 0.001 |
| Acetic acid | 0.008 |
| Propionic acid | 0.006 |
| Isobutyric acid | 0.018 |
| Butyric acid | 0.018 |
| Isovaleric acid | 0.01 |
| Valeric acid | 0.01 |
| Trans-3-hexanoic acid 1% DPG | 0.001 |
| 2-Methyl-4-pentanoic acid 1% DPG | 0.001 |
| Hexanoic acid | 0.001 |
| Octanoic acid | 0.1 |
| 2-Ethylhexanoic acid | 0.001 |
| Nonanoic acid | 0.001 |
| Decanoic acid | 0.001 |
| Undecanoic acid 10% DPG | 0.001 |
| Undecylenic acid 10% DPG | 0.001 |
| Dodecanoic acid | 0.5 |
| Tridecanoic acid | 0.3 |
| Myristic acid | 4 |
| Pentadecanoic acid | 3 |
| Palmitic acid | 11 |
| Palmitoleic acid | 0.01 |
| Heptadecanoic acid | 1.5 |
| Stearic acid | 1.5 |
| Oleic acid | 6 |
| Linolenic acid | 0.8 |
| Squalene | 36 |
| Cholesterol | 6 |
| Squalane | 1 |
| Triglyceride palmitate | 1 |
| Dipropylene glycol (DPG) | 100% in total |

### Masking effects of body odor and foot odor

Six hours after 10 males made a training run for about one hour in a closed 10m × 10m × 10m training room kept at 25°C and 65%HR and shed a sweat, 5 g of the body cosmetic composition of the present invention was applied to each of their both arms, neck, chest, back and both feet. Its masking effects rightly after application, two hours after application and after it was washed off were evaluated in accordance with the below-described criteria, with the sweat odor and foot odor before treatment being set at 5. Then, an average score was determined. The average score of 1 or greater but less than 2 was ranked A, that of 2 or greater but less than 3 was ranked B, that of 3 or greater but less than 4 was ranked C, and that of 4 or greater was ranked D.
1: The odor is masked completely.
2: The odor is substantially masked.
3: A slight sweat or foot odor remains.
4: A sweat or foot odor remains (deodorization or masking is a little insufficient).
5: A sweat or foot odor evidently remains (masking is insufficient).

### Example 1

Hair cosmetic compositions (deodorant compositions) as shown in Table 2 were prepared and their scalp-odor masking effects were measured.

**Table 2**

| | | | Invention products | | | | | | Comparative products | |
|---|---|---|---|---|---|---|---|---|---|---|
| | | | 1 | 2 | 3 | 4 | 5 | 6 | 1 | 2 |
| Components (%) | Betaine ester (1) | | 1 | 1 | - | - | - | - | - | - |
| | Betaine ester (2) | | - | - | 1 | 1 | - | - | - | - |
| | Deodorant composition* | | - | - | - | - | 1 | 1 | - | - |
| | Nylon powder | | 3 | 3 | 3 | 3 | 3 | 3 | 3 | 3 |
| | Styrene·stearyl methacrylate·divinyl benzene copolymer | | 0.1 | 0.1 | 0.1 | 0.1 | 0.1 | 0.1 | 0.1 | 0.1 |
| | Methylpolysiloxane ("Silicone KT-5, product of Toshiba Silicone) | | 1.5 | 1.5 | 1.5 | 1.5 | 1.5 | 1.5 | 1.5 | 1.5 |
| | Carboxyvinyl polymer ("Carbopole 981", product of B.F. Goodrich) | | 0.2 | 0.2 | 0.2 | 0.2 | 0.2 | 0.2 | 0.2 | 0.2 |
| | Potassium hydroxide | | 0.07 | 0.07 | 0.07 | 0.07 | 0.07 | 0.07 | 0.07 | 0.07 |
| | Dipotassium glycyrrhitinate | | 0.1 | 0.1 | 0.1 | 0.1 | 0.1 | 0.1 | 0.1 | 0.1 |
| | 1,3-Butylene glycol | | 1 | 1 | 1 | 1 | 1 | 1 | 1 | 1 |
| | Ethanol | | 50 | 50 | 50 | 50 | 50 | 50 | 50 | 50 |
| | Propyl paraoxybenzoate | | 0.1 | 0.1 | 0.1 | 0.1 | 0.1 | 0.1 | 0.1 | 0.1 |
| | Purified water | | Balance | Balance | Balance | Balance | Balance | Balance | Balance | Balance |
| | Perfume | | 0.1 | - | 0.1 | - | 0.1 | - | 0.1 | - |
| Deodorizing performance | Scalp odor | rightly after treatment | A | A | A | A | A | A | B | B |
| | | 30 min after treatment | A | A | A | A | A | A | B | C |
| | | 1 hr after treatment | A | A | A | A | A | A A | C | C |
| | | one day after treatment | B | B | B | B | B | B | D | D |

| | | | | | | | | | | |
|---|---|---|---|---|---|---|---|---|---|---|
| *) 80.0% of Betaine ester (1), 10.0% of cis-3-hexenol, 1.0% of β-ionone, 1.0% of α-damascone and 8.0% of geraniol. | | | | | | | | | | |

As shown in Table 2, any one of the invention products exhibited excellent effects for masking scalp odor.

### Example 2

Body cosmetic compositions as shown in Tables 3 and 4 were prepared and their deodorizing or masking effects of body odor or foot odor were measured.

**Table 3**

| | | | | Invention products | | | Comparative products |
|---|---|---|---|---|---|---|---|
| | | | 7 | 8 | 9 | 10 | 3 |
| Component (%) | Betaine ester (1) | | 1 | 1 | 1 | 1 | - |
| | Betaine ester (2) | | - | - | - | - | - |
| | Polyethylene glycol 200 | | 10 | 10 | 10 | 10 | 10 |
| | Polyethylene glycol 400 | | 30 | 30 | 30 | 30 | 30 |
| | Dipropylene glycol | | 4 | 4 | 4 | 4 | 4 |
| | 86% Glycerin | | 6 | 6 | 6 | 6 | 6 |
| | Polyoxyethylene (50) hydrogenated castor oil | | 0.3 | 0.3 | 0.3 | 0.3 | 0.3 |
| | Polyoxyethylene·methylpolysiloxane copolymer | | 0.6 | 0.6 | 0.6 | 0.6 | 0.6 |
| | Benenyl alcohol | | 2 | 2 | 2 | 2 | 2 |
| | Aluminum oxide | | 0.5 | 0.5 | 0.5 | 0.5 | 0.5 |
| | Methylpolysiloxane | | 8 | 8 | 8 | 8 | 8 |
| | Zeolite | | 20 | 20 | 20 | 20 | 20 |
| | Cyclodextrin | | 15 | 15 | 15 | 15 | 15 |
| | Hydroxyethane disulfonic acid | | 0.05 | 0.05 | 0.05 | 0.05 | 0.05 |
| | Disodium EDTA | | 0.01 | 0.01 | 0.01 | 0.01 | 0.01 |
| | Dibutylhydroxytoluene | | 0.1 | 0.1 | 0.1 | 0.1 | 0.1 |
| | pH Regulator | | q.s. | q.s. | q.s. | q.s. | q.s. |
| | Purified water | | Balance | Balance | Balance | Balance | Balance |
| | γ-Undecalactone | | - | 1 | - | - | - |
| | Magnol | | - | - | 1 | - | - |
| | Musk ketone | | - | - | - | 1 | - |
| Deodorizing performance | Sweat odor·foot odor | rightly after treatment | A | A | A | A | C |
| | | 2 hrs after treatment | B | A | A | A | C |
| | | after washing-off | B | A | A | A | C |

**Table 4**

| | | | Invention Products | | | | Comparative products |
|---|---|---|---|---|---|---|---|
| | | | 11 | 12 | 13 | 14 | 4 |
| Component (%) | Betaine ester (1) | | - | - | - | - | - |
| | Betaine ester (2) | | 1 | 1 | 1 | 1 | - |
| | Polyethylene glycol 200 | | 10 | 10 | 10 | 10 | 10 |
| | Polyethylene glycol 400 | | 30 | 30 | 30 | 30 | 30 |
| | Dipropylene glycol | | 4 | 4 | 4 | 4 | 4 |
| | 86% Glycerin | | 6 | 6 | 6 | 6 | 6 |
| | Polyoxyethylene (50) hydrogenated castor oil | | 0.3 | 0.3 | 0.3 | 0.3 | 0.3 |
| | Polyoxyethylene·methylpolysiloxane copolymer | | 0.6 | 0.6 | 0.6 | 0.6 | 0.6 |
| | Behenyl alcohol | | 2 | 2 | 2 | 2 | 2 |
| | Aluminum oxide | | 0.5 | 0.5 | 0.5 | 0.5 | 0.5 |
| | Methylpolysiloxane | | 8 | 8 | 8 | 8 | 8 |
| | Zeolite | | 20 | 20 | 20 | 20 | 20 |
| | Cyclodextrin | | 15 | 15 | 15 | 15 | 15 |
| | Hydroxyethane disulfonic acid | | 0.05 | 0.05 | 0.05 | 0.05 | 0.05 |
| | Disodium EDTA | | 0.01 | 0.01 | 0.01 | 0.01 | 0.01 |
| | Dibutylhydroxytoluene | | 0.1 | 0.1 | 0.1 | 0.1 | 0.1 |
| | pH Regulator | | q.s. | q.s. | q.s. | q.s. | q.s. |
| | Purified Water | | Balance | Balance | Balance | Balance | Balance |
| | γ-Undecalactone | | - | 1 | - | - | 1.0 |
| | Magnol | | - | - | 1 | - | - |
| | Musk ketone | | - | - | - | 1 | - |
| Deodorizing performance | Sweat odor foot odor | rightly after treatment | A | A | A | A | B |
| | | after two hours | B | A | A | A | C |
| | | after washing-off | B | A | A | A | C |

As apparent from Tables 3 and 4, the invention products each exhibited excellent deodorizing and masking effects against sweat odor and foot odor.

### Example 3 (Hair lotion)

| | % |
|---|---|
| Betaine ester (1) | 1 |
| Styrene·methyl methacrylate·dimethylpolysiloxane graft copolymer | 3.5 |
| Methylpolysiloxane ("Silicone KF-96A", product of Shin-etsu Silicone) | 1 |
| Acrylilc acid·alkyl methacrylate copolymer ("PEMULEN TR-1", product of B.F. Goodrich) | 0.2 |
| L-Arginine | 0.2 |
| dl-α-Tocopherol acetate | 0.1 |
| PEG600 | 1 |
| 1-Menthol | 0.3 |
| Ethanol | 3 |
| Ethyl paraoxybenzoate | 0.2 |
| Perfume composition A | 0.1 |
| Purified water | Balance to 100 |

### Composition of the perfume composition A:

| | % |
|---|---|
| Limonene | 15 |
| cis-3-Hexanol | 1 |
| Ligustral | 0.5 |
| Helional | 1.5 |
| Benzyl alcohol | 15 |
| Benzaldehyde | 0.1 |
| Lilial | 3 |
| Lyral | 2 |
| α-Damascone | 0.1 |
| Methyl dihydrojasmonate | 6 |
| Linalool | 2 |
| Benzyl salicylate | 5 |
| Coumarin | 0.1 |
| Vanillin | 0.2 |
| Tentalohm | 8 |
| β-ionone | 2 |
| γ-Undecalactone | 0.8 |
| cis-Jasmone | 0.2 |
| Dipropylene glycol | 19.5 |
| | Balance to 100 |

### Example 4 (Hair growth tonic)

| | % |
|---|---|
| Betaine ester (1) | 1 |
| Nicotinic acid amide | 0.1 |
| dl-α-Tocopherol acetate | 0.05 |
| β-glycyrrhetinic acid | 0.1 |
| *Swertiae Herba* extract | 1.2 |
| Carrot extract | 0.3 |
| St. John's wort extract | 0.5 |
| 1-Menthol | 0.2 |
| Extract of *Engelhardtia chrysolepis* Hance | 0.3 |
| Polyoxyethylene (25) hydrogenated castor oil | 0.2 |
| Stearyl trimethylammonium chloride ("QUARTAMIN 86W", product of Kao Corp.) | 0.5 |
| Ethanol | 50 |
| Perfume composition A | 0.1 |
| Purified water | Balance to 100 |
| CO² gas:propellant (stock/propellant) | 98/2 |

### Example 5 (Tonic)

| | % |
|---|---|
| Betaine ester (1) | 1 |
| Stearyl trimethylammonium chloride ("QUARTAMIN 86PC", product of Kao Corp.) | 0.2 |
| Polyoxyethylene (25) hydrogenated castor oil | 1 |
| Polyoxyethylene·polyoxypropylene stearyl ether | 0.7 |
| dl-α-Tocopherol acetate | 0.05 |
| Dipotassium glycyrrhitinate | 0.1 |
| Triclosan | 0.1 |
| Ethanol | 60 |
| Perfume composition A | 0.1 |
| Purified water | Balance to 100 |

### Example 6 (Shampoo composition)

| | % |
|---|---|
| Betaine ester (1) | 1 |
| Sodium polyoxyethylene (3) lauryl ether sulfate | 10 |
| Sodium lauryl sulfate | 5 |
| Polyoxyethylene (15) lauryl ether | 1 |
| Lauryl diethanolamide | 1 |
| Lauryl amidopropylbetaine | 1 |
| Stearyl trimethylammonium chloride | 0.1 |
| Perfume composition A | 0.4 |
| Purified water | Balance to 100 |

### Example 7 (Rinse)

| | % |
|---|---|
| Betaine ester (1) | 1 |
| Stearyl trimethylammonium chloride | 1.1 |
| Cetanol | 3 |
| Isopropyl palmitate | 0.5 |
| Dimethylpolysiloxane emulsion | 4 |
| Hydroxyethyl cellulose | 0.1 |
| Perfume composition A | 0.3 |
| Purified water | Balance to 100 |

### Example 8 (Spray)

| | % |
|---|---|
| Betaine ester (1) | 1 |
| Glycerin | 2 |
| Cetanol | 4 |
| Light liquid isoparaffin ("Isozole 400", product of Nippon Mitsubishi Corp.) | 4 |
| Polyoxyethylene (20) isocetyl ether | 0.5 |
| Isostearyl glyceryl ether | 2 |
| Dimethylpolysiloxane | 1 |
| 95% Ethanol | 85.4 |
| Perfume composition A | 0.1 |
| | Total: 100 |

### Example 9 (Hair mist)

| | % |
|---|---|
| Betaine ester (1) | 1 |
| Polyoxyethylene hydrogenated castor oil | 0.2 |
| Stearyl trimethylammonium chloride (28%) | 0.5 |
| "Softanol" | 0.1 |
| Glycerin | 3 |
| 95% Ethanol | 10 |
| Perfume composition A | 0.05 |
| Purified water | Balance to 100 |

### Example 10 (Hair cream)

| | % |
|---|---|
| Betaine ester (1) | 1 |
| Dimethylpolysiloxane | 5 |
| Sorbitol | 10 |
| α-Monoisostearyl glyceryl ether | 1 |
| Higher alcohol (oleyl alcohol) | 7 |
| Propylene glycol | 2 |
| 95% Ethanol | 2 |
| Polyoxyethylene sorbitan monooleate | 4 |
| Glycerol monostearate | 2 |
| Perfume composition A | 0.1 |
| Purified water A | Balance to 100 |

### Example 11 (hair growth tonic)

| | % |
|---|---|
| Betaine ester (1) | 1 |
| 95% Ethanol | 60 |
| 1-Menthol | 0.15 |
| Nicotinic acid amide | 0.15 |
| β-glycyrrhetinic acid | 0.12 |
| Swertianin | 1.5 |
| Extract of *Engelhardtia chrysolepis Hance* | 0.95 |
| St. John's wort extract | 0.09 |
| Touch feel improver | 0.2 |
| pH Regulator | 0.2 |
| Perfume composition A | 0.1 |
| Purified water | Balance to 100 |

### Example 12 (Hair growth tonic)

| | % |
|---|---|
| Betaine ester (1) | 1 |
| 95% Ethanol | 74 |
| 1-Menthol | 0.7 |
| Decyltetradecyl dimethylamine oxide | 0.15 |
| 1,3-Butanediol | 0.12 |
| Pantothenyl ethyl ether | 0.4 |
| Nicotinic acid amide | 0.1 |
| Benzyl nicotinate | 0.01 |
| β-Glycyrrhetinic acid | 0.2 |
| 1-Dodecene | 0.01 |
| Titanium dioxide | 0.5 |
| pH Regulator | 4.5 |
| Perfume composition A | 0.1 |
| Purified water | Balance to 100 |

### Example 13 (Hair liquid)

| | % |
|---|---|
| Betaine ester (1) | 1 |
| Acrylic amide·acrylilc acid·methacrylic methoxypolyethylene glycol copolymer | 2 |
| Polyoxypropylene (21) butyl ether phosphoric acid | 7 |
| Polyoxyethylene hydrogenated castor oil | 5 |
| Dipropylene glycol | 3 |
| Polyoxyethylene (20) isocetyl ether | 0.4 |
| Diethylene glycol monoethyl ether | 0.6 |
| 2-Hydroxo-4-methoxybenzophenone-5-sulfonic acid | 0.1 |
| Perfume composition A | 0.3 |
| Sodium hydroxide | 3 |
| 95% Ethanol | 60 |
| Buffer | 0.4 |
| Hair penetrant | 0.2 |
| Purified water | Balance to 100 |

The hair cosmetic compositions obtained in Examples 3 to 13 were found to have excellent masking and deodorizing effects of scalp odor.

### Example 14 (Medicated cream)

| | % |
|---|---|
| Betaine ester (2) | 1 |
| S-Allantoin (product of Kawasaki Fine Chemical) | 0.5 |
| N-(Hexadecydroxypropyl)-N-hydroxyethylhexadecanamide | 8 |
| Cholesteryl isostearate | 1 |
| Isostearyl glyceryl ether | 2 |
| Polyoxyethylene hydrogenated castor oil | 0.2 |
| Methylcyclopolysiloxane | 12 |
| Squalane | 4 |
| Neopentyl glycol dicaprate | 3 |
| Glyceryl monoisostearate monomyristate | 2 |
| Dimethylpalmitin polysiloxane | 1 |
| 86% Glycerin | 10 |
| Magnesium sulfate | 1 |
| Methyl paraoxybenzoate | 0.2 |
| Perfume composition B | 0.03 |
| Succinic acid | 2.5 |
| 48% sodium hydroxide | 1 |
| Purified water | Balance to 100 |

| Composition of Perfume composition B: | % |
|---|---|
| Hexyl acetate | 1.5 |
| Triplal | 0.9 |
| Helional | 0.3 |
| Magnol | 1.5 |
| Allyl aproate | 0.3 |
| Benzaldehyde (10% dipropylene glycol solution) | 0.6 |
| iso-Amyl acetate (10% dipropylene glycol solution) | 1.5 |
| Eucalyptol | 3 |
| Menthol | 18 |
| Mentha oil | 6 |
| Peppermint oil | 0.6 |
| Camphor | 3 |
| α-Pinene | 9 |
| iso-Bornyl acetate | 6 |
| Basil oil | 0.6 |
| Eugenol (10% dipropylene glycol solution) | 0.6 |
| Geraniol | 1.5 |
| Phenylethyl alcohol | 3 |
| Hedione | 9 |
| Jasmopyrane forte | 0.3 |
| Lilial | 3 |
| Linalol | 6 |
| Phenylacetaldehyde | 0.3 |
| Cedar wood | 1.5 |
| Ethylene brassylate | 3 |
| Pearlide (musk ketone) | 6 |
| Indole (10% dipropylene glycol solution) | 0.6 |
| Amyl salicylate | 6 |
| Propylene glycol | 6.4 |
| | Total: 100 |

### Example 15 (Body lotion)

| | % |
|---|---|
| Betaine ester (2) | 1 |
| Polyoxyethylene (50) hydrogenated castor oil | 0.7 |
| Triclosan | 0.2 |
| Bisabolol | 0.01 |
| Tetrahydroxybenzophenone | 0.04 |
| Triethyl citrate | 0.2 |
| Diisobutyl adipate | 0.15 |
| Isopropylene glycol | 0.05 |
| dl-Camphor | 0.02 |
| Menthol | 0.6 |
| Eucalyptus oil | 0.15 |
| Clove extract | 0.005 |
| Perfume composition B | 0.1 |
| 95% 8-acetylated sucrose-denatured alcohol | 50 |
| Disodium EDTA | 0.01 |
| Citric acid | 0.05 |
| Sodium citrate | 0.02 |
| Purified water | Balance to 100 |

### Example 16 (Gel-lotion)

| | % |
|---|---|
| Betaine ester (2) | 1 |
| Styrene·methyl methacrylate·dimethylpolysiloxane graft copolymer | 4 |
| Methylpolysiloxane | 0.5 |
| 95% 8-acetylated sucrose-denatured alcohol | 25 |
| 1,3-Butylene glycol | 1 |
| Polyethylene glycol 400 | 0.1 |
| Menthol | 0.1 |
| Isopropylmethylphenol | 0.1 |
| Methyl paraoxybenzoate | 0.1 |
| Perfume composition B | 0.1 |
| pH Regulator (sodium carbonate) | q.s. |
| Purified water | Balance to 100 |

### Example 17 (Body shampoo)

| | % |
|---|---|
| Betaine ester (1) | 1 |
| Triethanolamine N-lauryl-L-glutamate | 6 |
| N-Laurylmethyl taurine sodium | 3 |
| Triethanolamine laurate | 10 |
| Triethanolamine myristate | 10 |
| Lauryl imidazolinium betaine | 5 |
| Lauroyl diethanolamide | 5 |
| Propylene glycol | 7 |
| Perfume composition B | 0.5 |
| Antiseptic, chelating agent | q.s. |
| Purified water | Balance to 100 |

### Example 18 (Solid detergent)

| | % |
|---|---|
| Betaine ester (1) | 1 |
| Sodium Lauryl monoglyceride sulfate | 80 |
| Stearic monoglyceride | 7 |
| Cetyl alcohol | 10 |
| Perfume composition B | 1 |
| Purified water | Balance to 100 |

### Example 19 (Body compress)

| | % |
|---|---|
| Betaine ester (2) | 1 |
| Polyethylene glycol monolaurate (HLB: 13.7) | 8 |
| 86% Glycerin | 4 |
| 95% 8-acetylated sucrose-denatured alcohol | 15 |
| Menthol | 0.15 |
| Amino-modified silicone emulsion "SM8704C" | 1 |
| Perfume composition B | 0.02 |
| Antiseptic (methylparaben) | 0.05 |
| pH Regulator (48% sodium hydroxide) | q.s. |
| Purified water | Balance to 100 |

### Example 20 ("Moisturizing type" Skin lotion)

| | % |
|---|---|
| Betaine ester (2) | 2 |
| Succinic acid | 0.65 |
| Sodium hydrogenphosphate | 0.85 |
| Polyoxyethylene (20) isocetyl ether | 0.5 |
| Polyoxyethylene·methylpolysiloxane copolymer | 0.05 |
| Pullulan | 0.05 |
| 86% Glycerin | 8 |
| 1,3-Butylene glycol | 4 |
| Polyethylene glycol 1540 | 2.5 |
| Multitol | 0.2 |
| Methyl paraoxybenzoate | 0.1 |
| Hamamelis extract | 0.03 |
| Urea | 1 |
| ε-aminocaproic acid | 0.3 |
| L-Arginine | 0.3 |
| Trimethylglycine | 1 |
| Tuberose polysaccharide solution | 1 |
| Disodium EDTA | 0.2 |
| 95% 8-acetylated sucrose-denatured alcohol | 3 |
| Perfume composition B | 0.005 |
| Purified water | Balance to 100 |

### Example 21 (Powder type bath agent)

| | % |
|---|---|
| Betaine ester (1) | 1 |
| Sodium hydrogencarbonate | 49 |
| Colorant | q.s. |
| Perfume composition B | q.s. |
| Sodium sulfate | Balance to 100 |

### Example 22 (Tablet type bath agent)

| | |
|---|---|
| Betaine ester (1) | 1 |
| Sodium hydrogencarbonate | 25 |
| Sodium carbonate | 25 |
| Fumaric acid | 40 |
| Polyethylene glycol 400 | 5 |
| Magnesium oxide | 0.4 |
| Cyclodextrin | 0.5 |
| Calcium silicate | 0.1 |
| Perfume composition B | 0.5 |
| Colorant | q.s. |
| Purified water | Balance to 100 |

### Example 23 (Liquid type bath agent)

| | % |
|---|---|
| Betaine ester (1) | 1 |
| N-(Hexadecyloxyhydroxypropyl)-N-hydroxyethyldecanamide | 1.5 |
| Monochlesteryl alkenylsuccinate | 0.5 |
| Myristic acid | 0.5 |
| Isopropyl myristate | 0.5 |
| Glyceryl monoisostearate monomyristate | 0.5 |
| Liquid paraffin | 70 |
| Methylparaben | 0.3 |
| Rice bran oil ("Oryza Oil S-1", product of Ichimaru Pharcos) | 2 |
| Polyoxyethylene (6) sorbitan monostearate | 4 |
| Polyoxyethylene (20) sorbitan monooleate | 2 |
| Polyoxyethylene(40) sorbitol tetraoleate | 2 |
| Vegetable extract | 1 |
| Perfume composition B | 0.8 |
| Purified water | Balance to 100 |

### Example 24 (Liquid type bath agent)

| | % |
|---|---|
| Betaine ester (1) | 1 |
| Ethanol | 10 |
| Polyethylene glycol (molecular weight: 400) | 6.5 |
| Glycerin | 0.5 |
| Methylparaben | 0.1 |
| Liquid paraffin | 12.6 |
| Menthol | 0.5 |
| Phenoxyethanol | 0.1 |
| Polyoxyethylene (6) sorbitan monostearate | 5 |
| Polyoxyethylene (20) sorbitan monolaurate | 1 |
| Polyoxyethylene (20) sorbitan monostearate | 1 |
| Polyoxyethylene (20) sorbitan monooleate | 1 |
| Perfume composition B | 0.9 |
| Purified water | Balance to 100 |

The body cosmetic compositions in Examples 14 to 24 exhibited excellent masking and deodorizing effects against body odor and foot odor.

### Industrial Applicability

The compositions according to the present invention have excellent masking and deodorizing effects against scalp odor, body odor and foot odor.

## Claims

1. A deodorant comprising a betaine ester represented by the following formula (1): 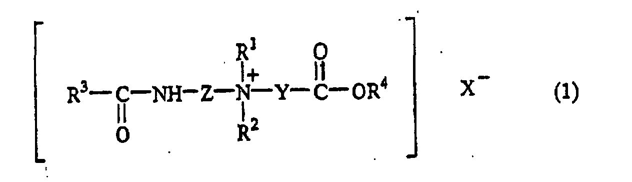 [wherein, R², R² and R³ each independently represents a hydrogen atom or a linear or branched C₁₋₃₆ alkyl or alkenyl group, Y represents a linear or branched C₁₋₄ alkylene group, Z represents a group represented by -R⁵(OA)ₙ- (in which, R⁵ represents a linear or branched C₁₋₃₆ alkylene group, A represents a linear or branched C₂₋₄ alkylene group and n stands for 0 to 10), R⁴ represents a residue left after removal of an OH from an alcohol compound having, in the molecule thereof, at least one hydroxyl group, and X⁻ represents an anion].

2. A deodorant composition of Claim 1, wherein the formula (1) is the following formula (2): 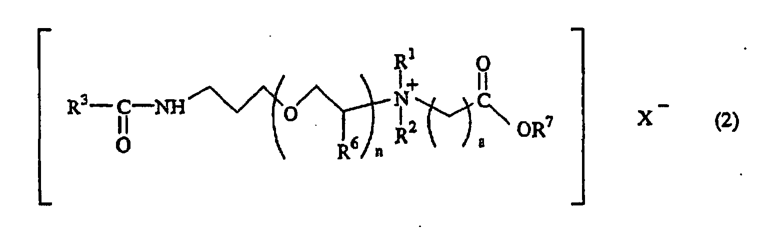 [wherein, R¹, R², R³, n and X⁻ have the same meanings as defined above, R⁶ represents a hydrogen atom or a methyl group, a stands for 1 or 2, R⁷ represents a saturated or unsaturated aliphatic hydrocarbon or aromatic hydrocarbon group having 1 to 11 carbon atoms, a C₁₂₋₃₆ alkadienyl, alkatrienyl, aryl or arylalkyl group, or a monocyclic, dicyclic or tricyclic terpene hydrocarbon group, some of the hydrogen atoms on said hydrocarbon group of R⁷ may be substituted by a halogen atom or a hydroxyl group, or the methylene group in said hydrocarbon group of R⁷ may be substituted by a carbonyl group, an amide bond, an oxygen atom or a sulfur atom, the methyl group may be substituted or unsubstituted by a formyl group or -CONH₂, the carbon-carbon double bond may be substituted by an epoxy group, or R⁷ may be either a single substance or a mixture].

3. A deodorant composition comprising the following components (a) and (b):
(a) a deodorant as claimed in Claim 1, and
(b) a functional perfume.

4. A hair cosmetic composition comprising a deodorant as claimed in Claim 1 or 2.

5. A hair cosmetic composition of Claim 4, containing the deodorant in an amount of 0.01 to 70 wt.%.

6. A hair cosmetic composition, comprising a deodorant composition of Claim 3 in an amount of 0.01 to 50 wt.%.

7. A body cosmetic composition, comprising a deodorant of Claim 1 or 2.

8. A body cosmetic composition of Claim 5, comprising a deodorant in an amount of 0.01 to 70 wt.%.
